# EUROPEAN PATENT SPECIFICATION

(11) **EP 3 697 373 B1**
(45) Date of publication and mention of the grant of the patent: **20.12.2023**
(21) Application number: 18867507.8
(22) Date of filing: 17.10.2018
(51) Int. Cl.: A61K 8/27, A61K 9/06, A61K 8/19, A61K 8/42, A61K 8/92, A61K 31/164, A61K 33/22, A61K 36/185, A61P 17/02, A61P 17/06, A61Q 19/00, A61K 8/31, A61K 33/30

(54) **PRODUCTION METHOD OF SKIN CARE CREAM**
HERSTELLUNGSVERFAHREN FÜR HAUTPFLEGECREME
PROCÉDÉ DE PRODUCTION D'UNE CRÈME DE SOINS POUR LA PEAU

(30) Priority: 17.10.2017 TR 201715943
(43) Date of publication of application: 26.08.2020
(73) Proprietor: Moss Farma Ilaç Itr. Med. Kozm. Tib. Cih. Ith. Ihr. San. Ve Tic. A.S., Ankara (TR)
(72) Inventor: CENIK, Mehmet, Ankara (TR)
(74) Representative: Yavuzcan, Alev
(86) International application number: PCT/TR2018/050603
(87) International publication number: WO 2019/078803

(56) References cited:
- WO-A1-01/30363
- WO-A1-2017/103936
- CN-A- 105 267 072
- CN-A- 106 266 384
- TR-A1- 201 515 631
- US-A1- 2004 013 744
- VISSCHER ET AL: "Update on the Use of Topical Agents in Neonates", NEWBORN AND INFANT NURSING REVIEWS, W.B. SAUNDERS, AMSTERDAM, NL, vol. 9, no. 1, 1 March 2009 (2009-03-01), pages 31-47, XP025907358, ISSN: 1527-3369, DOI: 10.1053/J.NAINR.2008.12.010 [retrieved on 2009-02-07]
- QUIRIN K W ET AL: "Sanddornlipide interessante Wirkstoffe fuer die Kosmetik", PARFUMERIE UND KOSMETIK, HUETHIG, HEIDELBERG, DE, vol. 74, no. 10, 1 January 1993 (1993-01-01), pages 618-625, XP009177992, ISSN: 0031-1952

## Description

### Technical Field

This invention is related to the production method of skin care cream, which can be used for treatment of irritation, redness, itch, dermatitis, scar, burn, skin dryness, allergy, eczema, skin crack, bedsore on the skin by revitalizing the skin cells without causing any side effect thanks to its formula.

Boric acid within the production formula of the skin care cream, which has antiseptic characteristic and decelerates reproduction of microbes on the skin, offers faster treatment process.

### Prior Art

Today, people prefer different creams for revitalization and treatment of the skin in the event of irritation, redness, itch, dermatitis, scar, burn, skin dryness, allergy, eczema, skin crack, bedsore occurring on the skin of women and men of any age, children or infants.

The Turkish Patent document numbered TR 2016/02911 developed to benefit from the cosmetic skin creams in a much more effective way explains the skin care cream and the skin care cream production method used for skin care, skin moisturizing, tightening, anti-wrinkling purposes and for treatment of atopic skins, fungal, infective or non-infective small scars, low-degree burns, fever blister, pregnancy cracks and rash within the scope of which water-solubilized pure propolis, pure aloe vera oil, pure shea butte oil, natural plant hormone unisteron- y50 and uber base cream are used.

The Turkish Patent document numbered TR 2016/11476 developed to benefit from the cosmetic skin creams in a much more effective way explains fully natural burn cream, which is capable of healing the first and second degree burns such as contact with flame or hot objects and sun burns without leaving any scar, except for the chemical burns and prevents rash and redness occurring on long-term bed-bound patients.

As it is explained above, the document included within the technique does not explain that the boric acid is available within the formula of the cream and thereby, ensures faster skin treatment.

### Detailed description of the invention

The present invention is the production method of skin care cream comprising of following production steps;
- preparation of the coconut oil in the rate between 1% and 4%, hippophae oil in the rate between 1% and 5%, vaseline between 28% and 37%, liquid vaseline between 17% and 21%, lanolin oil between 19% and 26% and finally wax in the rate between 3% and 6% before start of the production,
- melting the preparation under 70 centigrade degrees within a boiler,
- cooling the melted preparation under 50 centigrade degrees,
- adding zinc oxide with a percentage between 6% and 10%, DexPanthenol with a rate between 3% and 6% and boric acid in the rate between 1% and 6% and mixing the preparation until homogeneous appearance is obtained,
- adding wax in the rate between 3% and 6% to the mixture,
- adding phloramin as an odorant in the rate between 1% and 4% to the mixture,
- packaging the product.

Boric acid substance added in the rate between 1% and 6% to the content of the production method formula of the skin care cream of the present invention is an agent, which has antiseptic characteristic and decelerates reproduction of microbes on the skin.

The coconut oil included within the content of the production method formula of the skin care cream of the present invention nourishes the skin and assists in protection against infections. Furthermore, Palmitoleic (Omega 7), which is active substance of the hippophae oil, is a source of healing within the scope of skin diseases and Omega 7 contributes to prevention of extreme dryness on the skin due to the fact that Omega 7 enhances water-holding capacity of the body.

The vaseline and liquid vaseline substance included within the content of the production method formula of the skin care cream of the present invention has anti-bacterial characteristics and is used for treatment of skin dryness, skin burns and skin scars. The lanolin oil within the formula prevents loss of moisture. Its impact is bidirectional; whereas, wax contributes to renewal of the dry skin and scar pains thanks to its water texture.

The zinc oxide substance included within the content of the production method formula of the skin care cream of the present invention is an extremely important vitamin for the skin. It enhances the immunity. Furthermore, it ensures generation of new cells and improves scar healing in the event of missing zinc content in the skin. The zinc within the formula acts as a complimentary component for scar healing.

The wax substance included within the content of the production method formula of the skin care cream of the present invention is capable of solidifying and contributes to protection of components. DexPanthenol substance added to the mixture repairs the skin and thereby, renews the skin. Finally, phloramin added to the mixture formula adds odor to the final product.

The cream produced via the production method of the skin care cream of the present invention does not cause any side effect on the skin and its formula content creates barrier on the skin before occurrence of rash.

## Claims

1. Production method of skin care cream **characterized in that** the present invention comprises of following production steps;
preparation of the coconut oil in the rate between 1 % and 4%, hippophae oil in the rate between 1 % and 5%, vaseline between 28% and 37%, liquid vaseline between 17% and 21 %, lanolin oil between 19% and 26% and finally wax in the rate between 3% and 6% before start of the production,
melting the preparation under 70 centigrade degrees within a boiler, cooling the melted preparation under 50 centigrade degrees, adding zinc oxide with a percentage between 6% and 10%, DexPanthenol with a rate between 3% and 6% and boric acid in the rate between 1 % and 6% and mixing the preparation until homogeneous appearance is obtained,
adding wax in the rate between 3% and 6% to the mixture, adding phloramin as an odorant in the rate between 1 % and 4% to the mixture, packaging the product.

## Patentansprüche

1. Verfahren zur Herstellung einer Hautpflegecreme, **dadurch gekennzeichnet, dass** die vorliegende Erfindung die folgenden Herstellungsschritte umfasst;
Zubereitung des Kokosöls in einem Anteil zwischen 1 % und 4 %, Hippophae-Öl in einem Anteil zwischen 1 % und 5 %, Vaseline zwischen 28 % und 37 %, flüssige Vaseline zwischen 17 % und 21 %. Lanolinöl zwischen 19 % und 26 % und schließlich Wachs in einem Anteil zwischen 3 % und 6 % vor Beginn der Produktion.
Schmelzen des Präparats unter 70 Grad Celsius in einem Kessel, Abkühlen des geschmolzenen Präparats unter 50 Grad Celsius, Hinzufügen von Zinkoxid mit einem Anteil zwischen 6% und 10%. DexPanthcnol mit einem Anteil zwischen 3 % und 6 % und Borsäure in einem Anteil zwischen 1 % und 6 % zugeben und die Zubereitung mischen, bis ein homogenes Aussehen erreicht ist.
Zugabe von Wachs in einem Anteil zwischen 3 % und 6 % zu der Mischung, Zugabe von Phloramin als Geruchsstoff in einem Anteil zwischen
1 % bis 4 % der Mischung, Verpacken des Produkts.

## Revendications

1. Méthode de production d'une crème de soin de la peau **caractérisée par le fait que** la présente invention comprend les étapes de production suivantes ;
préparation de l'huile de coco à raison de 1 % à 4 %, de l'huile d'hippophae à raison de 1 % à 5 %, de la vaseline à raison de 28 % à 37 %, de la vaseline liquide à raison de 17 % à 21 %, de l'huile de lanoline à raison de 19 % à 26 % et enfin de la cire à raison de 3 % à 6 %. De l'huile de lanoline entre 19% et 26% et enfin de la cire à raison de 3% à 6% avant le début de la production.
faire fondre la préparation à 70 degrés centigrades dans une chaudière, refroidir la préparation fondue à 50 degrés centigrades, ajouter de l'oxyde de zinc avec un pourcentage compris entre 6% et 10%. DexPanthcnol à un taux compris entre 3% et 6% et de l'acide borique à un taux compris entre 1% et 6% et en mélangeant la préparation jusqu'à l'obtention d'un aspect homogène.
